# EUROPEAN PATENT APPLICATION

(11) **EP 1 992 603 A1**
(43) Date of publication of application: **19.11.2008**
(21) Application number: 07715059.7
(22) Date of filing: 28.02.2007
(51) Int. Cl.: C07C 19/16, C07C 17/278

(54) **TERMINALLY IODIZED POLYFLUOROALKANE OLIGOMER AND METHOD FOR PRODUCING SAME**

(30) Priority: 28.02.2006 JP 2006052380; 09.03.2006 JP 2006063510
(71) Applicant: Unimatec Co., Ltd., Tokyo 105-8585 (JP)
(72) Inventor: HORIUTI, Masayosi, Kitaibaraki-shi, Ibaraki 3191544 (JP); MURATA, Seiichiro, Kitaibaraki-shi, Ibaraki 3191544 (JP); SATO, Katsuyuki, Kitaibaraki-shi, Ibaraki 3191544 (JP); ABE, Hideki, Kitaibaraki-shi, Ibaraki 3191544 (JP)
(74) Representative: Adam, Holger
(86) International application number: PCT/JP2007/053773
(87) International publication number: WO 2007/102371

(57) **Abstract**

A terminally iodized polyfluoroalkane oligomer represented by the following general formula :

CₙF₂ₙ₊₁(CH₂CF₂)ₛ₊ₚ(CF₂CF₂)ₜ₊ᵣI [I]

(wherein n is an integer of 1 to 6, s+p is an integer of 1 to 4, showing the number of vinylidene fluoride skeleton, and t+r is an integer of 1 to 6, showing the number of tetrafluoroethylene skeleton, the adjacent group to the CₙF₂ₙ₊₁ group being the CH₂CF₂ group) can be produced by reaction of a terminally iodized polyfluoroalkane, represented by the following general formula :

CₙF₂ₙ₊₁(CH₂CF₂)ₛ₊ₚ(CF₂CF₂)ₜI

with tetrafluoroethylene in the presence of a peroxide initiator.

## Description

### TECHNICAL FIELD

The present invention relates to a terminally iodized polyfluoroalkane oligomer and a process for producing the same, and more particularly to a terminally iodized polyfluoroalkane oligomer obtained by oligomeric addition of vinylidene fluoride and tetrafluoroethylene, and a process for producing the same.

### BACKGROUND ART

A process for telomerization of perfluoroalkyl iodide as a starting material with vinylidene fluoride in the presence of a metal or a metal complex as a catalyst is well known. Furthermore, a process for conducting the telomerization in the presence of a radical generating agent at elevated temperatures, under high pressure, or under both conditions of elevated temperatures and high pressure is also well known. The reaction at elevated temperatures under high pressure requires a large amount of energy, and also hydrofluoric acid, etc. generated at the time of reaction cause vigorous corrosion of apparatuses, thereby increasing the renewal frequency of apparatuses, whereas in the case of using corrosion-resistant materials the apparatus cost will be inevitably increased due to higher cost of the materials.
Patent Literature 1 : JP-A-60-106533
Patent Literature 2 : JP-A-60-104023
Non-Patent Literature 1 : J. Fluorine Chem., 70, 215 (1995)
Non-Patent Literature 2 : J. Fluorine Chem., 102, 253 (2000)

It has been reported that compounds, in which the terminal perfluoroalkyl groups of the resulting telomer have about 8 carbon atoms, a high degree of biological accumulation, i.e. a serious environmental problem, resulting in a fear of less prospective future production and use thereof. However, compounds, whose perfluoroalkyl groups have 6 or less carbon atoms, are regarded as having a low degree of biological accumulation.

### DISCLOSURE OF THE INVENTION

### PROBLEM TO BE SOLVED BY THE INVENTION

An object of the present invention is to provide a terminally iodized polyfluoroalkane oligomer obtained by oligomeric addition of vinylidene fluoride and tetrafluoroethylene, which is such a compound whose perfluoroalkyl group has not more than 6 carbon atoms as regarded as having a low degree of biological accumulation, and also a process for producing the same.

### MEANS FOR SOLVING THE PROBLEM

The present invention provides a terminally iodized polyfluoroalkane oligomer represented by the following general formula :

CₙF₂ₙ₊₁(CH₂CF₂)ₛ₊ₚ(CF₂CF₂)ₜ₊ᵣI [I]

(where n is an integer of 1 to 6, s+p is an integer of 1 to 4, showing the number of vinylidene fluoride skeleton, t+r is an integer of 1 to 6, showing the number of tetrafluoroethylene skeleton, and the adjacent group to the CₙF₂ₙ₊₁ is the CH₂CF₂ group), which may be generally in the form of a mixture of oligomers having a variety in integers of s+p and t+r within the afore-defined ranges.

The terminally iodized polyfluoroalkane oligomer can be produced by reaction of a terminally iodized polyfluoroalkane represented by the following general formula :

CₙF₂ₙ₊₁(CH₂CF₂)ₛ₊ₚ(CF₂CF₂)ₜI [II]

(where n is an integer of 1 to 6, s+p is an integer of 1 to 4, showing the number of vinylidene fluoride skeleton, and t is an integer of 0 to 3, showing the number of tetrafluoroethylene skeleton in the raw material) with tetrafluoroethylene in the presence of a peroxide initiator, to obtain the terminally iodized polyfluoroalkane oligomer represented by the following general formula :

CₙF₂ₙ₊₁(CH₂CF₂)ₛ₊ₚ(CF₂CF₂)ₜ₊ᵣI [I]

(where n, s+p, and t are as defined above, and r is an integer of 1 to 5, showing the number of tetrafluoroethylene skeleton as added by the reaction, but t+r is an integer of 1 to 6). The compound [II] can be used in the form of a mixture of compounds having a variety in integers of n, s+p, and t within the above-defined ranges.

### EFFECT OF THE INVENTION

The present terminally iodized polyfluoroalkane oligomer has not only a perfluoroalkyl group of not more than 6 carbon atoms, which are regarded as having a low degree of biological accumulation, but also a CH₂CF₂ groups derived from vinylidene fluoride in the molecule, so double bonds, which are very susceptible to decomposition by ozone, can be easily formed by dehydrofluorination (HF removal), thereby causing less environmental hazard.

From the viewpoint of uses of the present terminally iodized polyfluoroalkane oligomer, the terminal iodide group can be converted to a terminal-OH group, which can be made to react with (meth)acrylic acid to form a terminal (meth)acrylic acid ester. These conversion products can be further formed into surfactants, water- and oil-repellents, surface-modifying agent, etc., so the present terminally iodized polyfluoroalkane oligomer can be effectively used as a raw material for synthesizing these derivative compounds.

### BEST MODES FOR CARRYING OUT THE INVENTION

Terminally iodized polyfluoroalkane [II] as a raw material for synthesizing a terminally iodized polyfluoroalkane oligomer [I] can be obtained by making a perfluoroalkyl group-containing iodide compound represented by the following general formula :

CₙF₂ₙ₊₁(CH₂CF₂)ₛI [III]

(where n : an integer of 1-6, and s : an integer of 0-3, showing the number of vinylidene fluoride skeleton in the raw material compound) to react with vinylidene fluoride in the presence of a peroxide initiator, thereby obtaining a terminally iodized polyfluoroalkane represented by the following general formula :

CₙF₂ₙ₊₁(CH₂CF₂)ₛ₊ₚI [IV]

(where s+p : an integer of 1-4, showing the number of vinylidene fluoride skeleton, but p being an integer of 1-3, showing the number of vinylidene fluoride skeleton as added by the reaction), and by making the resulting terminally iodized polyfluoroalkane [IV] as a starting material to react with tetrafluoroethylene in the presence of a peroxide initiator. The compound [III] or [IV] can be used in the form of a mixture of compounds having a variety of integers of n, s, and s+p within the above-defined ranges.

The addition reaction of the vinylidene fluoride and the tetrafluoroethylene is carried out in the presence of a peroxide initiator, which includes, for example, t-butyl peroxide, (t-butylcyclohexyl) peroxydicarbonate, dicetyl peroxydicarbonate, di-n-propyl peroxydicarbonate, diisopropyl peroxydicarbonate, di-t-butyl peroxydicarbonate, etc. From the viewpoint of reaction progress and controllability, about 0.05 to about 0.5% by mole, preferably about 0.1 to about 0.25% by mole, of the organic peroxide initiator can be used on the basis of the terminally iodized compound [III] or [IV].

Specific perfluoroalkyl group-containing iodide compound [III] includes, for example, the following compounds :

CF₃I

CF₃CF₂I

CF₃(CF₂)₂I

CF₃(CF₂)₃I

CF₃(CF₂)₄I

CF₃(CF₂)₅I

CF₃(CH₂CF₂)I

C₂F₅(CH₂CF₂)I

C₂F₅(CH₂CF₂)₂I

C₃F₇(CH₂CF₂)I

C₃F₇CH₂CF₂)₂I

C₄F₉(CH₂CF₂)I

C₄F₉(CH₂CF₂)₂I

The vinylidene fluoride addition reaction product [IV] is a mixture of oligomers having a variety in integers of s+p, where a desired vinylidene fluoride addition reaction product having a specific integer of s+p can be isolated from the mixture by distillation. Undesired oligomers having no specific integers of s+p can be reused in the oligomer-growing reaction with vinylidene fluoride upon isolation or as in the form of the mixture.

The desired product of the present invention, i.e. terminally iodized polyfluoroalkane oligomer [I] can be obtained by making the terminally iodized polyfluoroalkane [II] as a raw material for synthesizing the oligomer [I] to react with tetrafluoroethylene in the presence of a peroxide initiator. The peroxide initiator for use in the reaction can be the same organic peroxide initiator as mentioned above in the same proportion as mentioned above.

Reaction temperature of the addition reaction of vinylidene fluoride and tetrafluoroethylene depends on the decomposition temperature of the initiator to be used, and the reaction can be carried out at 80°C or lower even under low pressure by using a peroxide initiator decomposable at low temperatures. To conduct reaction at low temperatures means reduction not only in energy consumption, but also in suppression of apparatus corrosion by hydrofluoric acid, etc., and reduction of apparatus renewal frequency, and furthermore possible use of much cheaper apparatus materials, which together with the reduction in renewal frequency, can lead to suppression of capital investment to a lower cost.

Addition reaction of vinylidene fluoride and tetrafluoroethylene can be carried out by charging perfluoroalkyl group-containing iodide compound [III] or terminally iodized polyfluoroalkane [II] (which will be [IV] at t=0) into an autoclave, elevating the internal temperature to about 10° to about 60°C, for example, 50°C, then adding thereto a solution of a peroxide initiator in perfluoroalkyl group-containing iodide compound [III] or terminally iodized polyfluoroalkane [II], adding vinylidene fluoride or tetrafluoroethylene portion-wise thereto, while keeping the pressure at about 0.1 to about 10MPa, when the internal temperature reaches, for example, 55°C, and aging the reaction mixture at a temperature, for example, between about 55° to about 80°C for one hour after the portion-wise added amount reaches a desired one. Integer p of vinylidene fluoride skeleton or integer r of tetrafluoroethylene skeleton as added by the reaction depends on the added amount thereof, and generally a mixture of products having a variety in integers of s+p or t+r can be formed thereby.

Specific terminally iodized polyfluoroalkane oligomer [I] as an ultimate product includes, for example, the following compounds :
C₂F₅(CH₂CF₂)(CF₂CF₂)I
C₂F₅(CH₂CF₂)(CF₂CF₂)₂I
C₂F₅(CH₂CF₂)₂(CF₂CF₂)I
C₂F₅(CH₂CF₂)₂(CF₂CF₂)₂I
C₄F₉(CH₂CF₂)(CF₂CF₂)I
C₄F₉(CH₂CF₂)₂(CF₂CF₂)I
C₄F₉(CH₂CF₂)(CF₂CF₂)₂I
C₄F₉(CH₂CF₂)₂(CF₂CF₂)₂I

Tetrafluoroethylene addition reaction product [I] is a mixture of oligomers having a variety in integer of t+r, and an desired oligomer having a specific integer of t+r can be isolated by distillation of the mixture. Undesired oligomers having no specific integers of t+r can be reused in the oligomer-growing reaction with tetrafluoroethtylene upon isolation or as in the form of the mixture as such.

### EXAMPLES

The present invention will be described in detail below, referring to Examples.

### EXAMPLE 1

500g of perfluorobutyl iodide C₄F₉I (purity : 82.9%) was charged into an autoclave having a capacity of 1,200ml and when the internal temperature was elevated to 50°C, a solution of 0.75g (0.13mol.%) of di(4-t-butylcyclohexyl) peroxydicarbonate initiator (Percadox 16, a product of Kayaku Akzo Co.,) in 60g of C₄F₉I was added thereto. When the internal temperature reached to 55°C, vinylidene fluoride was portion-wise added thereto, while keeping the pressure at 0.5-0.7MPa, and after the portion-wise added amount reached to 214g, aging was conducted at 55° -65°C for one hour to complete the reaction. Then, the reaction mixture was cooled to recover 583g of the product.

The resulting product was distilled under conditions of column top temperature : 58°C, and pressure : 7.4kPa (56mmHg) to isolate 203g of CF₃(CF₂)₃(CH₂CF₂)I (purity : 99.5%), which was used as a raw material for reaction in the following Examples 2 and 3. Reaction product CF₃(CF₂)₃(CH₂CF₂)₂I was isolated by distillation under conditions of column top temperature : 74°C and pressure : 2.6kPa (20mmHg).

### EXAMPLE 2

600g of CF₃(CF₂)₃(CH₂CF₂)I (purity : 99.5%) was charged into an autoclave having a capacity of 1,200ml, and when the internal pressure was elevated to 50°C, a solution of 1.35g (0.15mol.%) of a peroxide initiator (Percadox 16) in 300g of CF₃(CF₂)₃(CH₂CF₂)I was added thereto. When the internal temperature reached to 55°C, tetrafluoroethylene was portion-wise added thereto, while keeping the pressure at 0.2-0.3MPa. After the portion-wise added amount reached to 150g, aging was carried out at 55° - 74°C for one hour to complete the reaction. Then, the reaction mixture was cooled to recover 1,010g of the product.

The resulting product was distilled under conditions of column top temperature : 71°C and pressure : 2.6kPa (20mmHg) to isolate 347g of CF₃(CF₂)₃(CH₂CF₂)(CF₂CF₂)I (purity : 99.8%), which was also used as a raw material for reaction in the following Example 3.

### EXAMPLE 3

830g of a mixture of CF₃(CF₂)₃(CH₂CF₂)I (purity : 99.5%) and CF₃(CF₂ₕ(CH₂CF₂)(CF₂CF₂)I (purity : 99.8%) (weight ratio 35.4 : 64.0) was charged into an autoclave having a capacity of 1,200ml, and when the internal temperature was elevated to 50°C, a solution of 1.68g (0.18mol.%) of a peroxide initiator (Percadox 16) in 300g of the above-mentioned mixture of the same composition ratio as above was added thereto. When the internal temperature reached to 55°C, tetrafluoroethylene was portion-wise added thereto, while keeping the pressure at 0.2-0.3MPa. After the portion-wise added amount reached to 150g, aging was carried out at 55° - 78°C for one hour to complete the reaction. Then, the reaction mixture was cooled to recover 1,257g of product mixture.

The resulting product was distilled to isolate 184g of CF₃(CF₂)₃(CH₂CF₂)I (purity : 99.7%), 575g of CF₃(CF₂)₃(CH₂CF₂)(CF₂CF₂)I (purity : 99.4%), and 302g of CF₃(CF₂)₃(CH₂CF₂)(CF₂CF₂)₂I (purity : 99.3%). Isolation of CF₃(CF₂)₃(CH₂CF₂)I and CF₃(CF₂)₃(CH₂CF₂)(CF₂CF₂)I by distillation was carried out under the same distillation conditions as above, whereas that of CF₃(CF₂)₃(CH₂CF₂)(CF₂CF₂)₂I was carried out under the distillation conditions of column top temperature : 91°C and pressure : 0.8kPa (6.0mmHg).

Analytical results by gas chromatography (GC) of the products obtained in the foregoing Examples are shown in the following Table 1 as GC% of compounds represented by the following general formula having a variety in integers of n, s(+p), and t(+r).

CₙF₂ₙ₊₁(CH₂CF₂)ₛ₍₊ₚ₎(CF₂CF₂)ₜ₍₊ᵣ₎I

**Table 1**

| | | | Example 1 | | Example 2 | | Example 3 | |
|---|---|---|---|---|---|---|---|---|
| n | s(+p) | t(+r) | Raw material | Product | Raw material | Product | Raw material | Product |
| 4 | 0 | 0 | 82.9 | 5.6 | | | | |
| 4 | 1 | 0 | | 61.4 | 99.5 | 44.7 | 35.4 | 14.8 |
| 4 | 2 | 0 | | 20.2 | | | | |
| 4 | 3 | 0 | | 2.4 | | | | |
| 4 | 4 | 0 | | 0.2 | | | | |
| 4 | 1 | 1 | | | | 37.1 | 64.0 | 46.2 |
| 4 | 1 | 2 | | | | 12.0 | | 23.9 |
| 4 | 1 | 3 | | | | 3.5 | | 9.3 |
| 4 | 1 | 4 | | | | 0.8 | | 3.0 |
| 4 | 1 | 5 | | | | 0.2 | | 0.8 |
| 4 | 1 | 6 | | | | | | 0.2 |
| C₆F₁₃H | | | 16.7 | 7.9 | | | | |

| | | | | | | | | |
|---|---|---|---|---|---|---|---|---|
| Note : C₆F₁₃H(CF₃CF₂CF₂CF₂CF₂CF₂H) is an impurity contained in the raw material C₄F₉I and is hard to isolate, because its boiling point is so close to that of C₄F₉I. Therefore, C₆F₁₃H is used in the succeeding reaction without isolation, because it does not take part in the succeeding reaction. | | | | | | | | |

### EXAMPLE 4

1,000g of perfluorobutyl iodide C₄F₉I (purity : 82.9%) and 1.5g (0.15mol.%) of di(4-t-butylcyclohexyl) peroxydicarbonate initiator (Percadox 16) were charged into an autoclave having a capacity of 1,200ml, and when the internal temperature was elevated to 55°C, vinylidene fluoride was portion-wise added thereto, while keeping the pressure at 0.4-0.8MPa. After the portion-wise added amount reached to 210g, aging was carried out at 55° -67°C for one hour to complete the reaction. Then, the reaction mixture was cooled to recover 1,097g of the product having a composition the shown in the following Table 2.

### EXAMPLE 5

500g of a mixture of distillate of the recovered product of Example 4 and perfluorobutyl iodide C₄F₉I (purity : 82.9%) (for the composition, see the following Table 2), and 0.6g (0.13mol.%) of di(4-t-butylcyclohexyl) peroxydicarbonate initiator (Percadox 16) were charged into an autoclave having a capacity of 1,200ml, and when the internal temperature was elevated to 55°C, vinylidene fluoride was portion-wise added thereto, while keeping the pressure at 0.4-0.8MPa. After the portion-wise added amount reached to 203g, aging was carried out at 55° -65°C for one hour to complete the reaction. Then, the reaction mixture was cooled to recover 606g of the product having a composition shown in the following Table 2.

### EXAMPLE 6

8,000g of perfluoroethyl iodide C₂F₅I (purity : 97.8%) and 30g (0.24mol.%) of di(4-t-butylcyclohexyl) peroxydicarbonate initiator (Percadox 16) were charged into an autoclave having a capacity of 10L, and when the internal temperature was elevated to 55°C, vinylidene fluoride was portion-wise added thereto, while keeping the pressure at 0.8-1.0MPa. After the portion-wise added amount reached to 764g, aging was carried out at 55° - 75°C for one hour to complete the reaction. Then, the reaction mixture was cooled to recover 7,845g of the product having a composition shown in the following Table 2.

### EXAMPLE 7

424kg of perfluoroalkyl iodides (a mixture of C₂F₅I, C₄F₉I, and C₆F₁₃I having a composition shown in the following Table 2), and 0.6kg (0.14mol.%) of di(4-t-butylcyclohexyl) peroxydicarbonate initiator (Percadox 16) were charged into an autoclave having a capacity of 300L, and when the internal temperature was elevated to 50°C, vinylidene fluoride was portion-wise added thereto, while keeping the pressure at 0.2-0.5MPa. After the portion-wise added amount reached to 78kg, aging was carried out at 50° -60°C for one hour to complete the reaction. Then, the reaction mixture was cooled to recover 477kg of the product having a composition shown in the following Table 2.

Analytical results by GC of the products obtained in the foregoing Examples 4 to 7 are shown by GC% of compounds represented by the following general formula having a variety in integers of n, and s(+p) in the following Table 2.

CₙF₂ₙ₊₁(CH₂CF₂)ₛ₍₊ₚ₎I

**Table 2**

| | | Example 4 | | Example 5 | | Example 6 | | Example 7 | |
|---|---|---|---|---|---|---|---|---|---|
| n | s(+p) | Raw material | Recovered product | Raw material | Recovered product | Raw material | Recovered product | Raw material | Recovered product |
| 2 | 0 | | | | | 99.0 | 77.3 | 3.6 | 1.3 |
| 4 | 0 | 82.9 | 6.1 | 77.1 | 0.8 | | | 78.2 | 10.5 |
| 6 | 0 | | | | | | | 8.4 | 1.0 |
| 2 | 1 | | | | | | 17.1 | | 2.0 |
| 4 | 1 | | 60.9 | 5.6 | 40.3 | | | | 61.5 |
| 6 | 1 | | | | | | | | 5.4 |
| 2 | 2 | | | | | | 3.8 | | 0.5 |
| 4 | 2 | | 19.4 | | 35.2 | | | | 9.6 |
| 6 | 2 | | | | | | | | 0.5 |
| 2 | 3 | | | | | | 0.5 | | 0.2 |
| 4 | 3 | | 2.3 | | 10.0 | | | | 0.5 |
| 6 | 3 | | | | | | | | 0.2 |

## Claims

1. (Amended) A terminally iodized polyfluoroalkane oligomer, represented by the following general formula :
CₙF₂ₙ₊₁(CH₂CF₂)ₛ₊ₚ(CF₂CF₂)ₜ₊ᵣI [I]
(where n is an integer of 1 to 6, s+p is an integer of 1 to 3, showing the number of vinylidene fluoride skeleton, and t+r is an integer of 1 to 6, showing the number of tetrafluoroethylene skeleton, the adjacent group to the CₙF₂ₙ₊₁ group being the CH₂CF₂ group).

2. A terminally iodized polyfluoroalkane oligomer according to Claim 1, which is a mixture of the oligomer having a variety in integers of s+p and t+r.

3. (Amended) A process for producing a terminally iodized polyfluoroalkane oligomer, represented by the following general formula :
CₙF₂ₙ₊₁(CH₂CF₂)ₛ₊ₚ(CF₂CF₂)ₜ₊ᵣI [I]
(where n and s+p have the same meanings as defined below, r is an integer of 1 to 5, showing the number of tetrafluoroethylene skeleton added by reaction, but t+r is an integer of 1 to 6), **characterized by** allowing a terminally iodized polyfluoroalkane, represented by the following general formula :
CₙF₂ₙ₊₁(CH₂CF₂)ₛ₊ₚ(CF₂CF₂)ₜI [II]
(where n is an integer of 1 to 6, s+p is an integer of 1 to 3, showing the number of vinylidene fluoride skeleton, and t is an integer of 0 to 3, showing the number of tetrafluoroethylene skeleton in the raw material) to react with tetrafluoroethylene in the presence of a peroxide initiator.

4. A process for producing a terminally iodized polyfluoroalkane oligomer according to Claim 3, wherein the compound [II] is a mixture of compounds having a variety in integers of n, s+p, and t.

5. A process for producing a terminally iodized polyfluoroalkane oligomer according to Claim 3, wherein the reaction is carried out at a temperature of not higher than 80°C.

6. (Cancelled)

7. (Cancelled)

8. (Cancelled)
